# EUROPÄISCHE PATENTANMELDUNG

(11) **EP 2 574 659 A1**
(43) Veröffentlichungstag der Anmeldung: **03.04.2013**
(21) Anmeldenummer: 12186021.7
(22) Anmeldetag: 26.09.2012
(51) Int. Cl.: C12M 1/107

(54) **Biogasreaktor mit einem Reaktorbehälter**

(30) Priorität: 29.09.2011 DE 202011106472 U
(71) Anmelder: 4Biogas GmbH & Co. KG, 44229 Dortmund (DE)
(72) Erfinder: Brachthäuser, Benno, 44229 Dortmund (DE); Kiomall, Daniel, 44137 Dortmund (DE); Karl, Rainer, 99718 Clingen (DE); Bexten, Matthias, 33378 Rheda-Wiedenbrück (DE)
(74) Vertreter: Meinke, Dabringhaus und Partner GbR

(57) **Zusammenfassung**

Die Erfindung betrifft einen Biogasreaktor (1) mit einem Hauptreaktorbehälter (2).

Um einen Biogasreaktor (1) zur Verfügung zu stellen, der bei einem Einsatz von 80 Volumenprozent Gülle elektrische Energie von mehr als 60 kW erzeugt, schlägt die Erfindung vor, dass der Durchmesser D des Reaktors (1) zwischen 4,2 m und 5 m und die Gesamthöhe H des Reaktors (1) zwischen 15,5 m und 18 m beträgt.

## Beschreibung

Die Erfindung betrifft einen Biogasreaktor mit einem Hauptreaktorbehälter.

Die Druckschrift DE 202 19 144 U1 offenbart einen Biogasreaktor zur Aufbereitung von pflanzlicher und tierischer Biomasse. Dieser bekannte Biogasreaktor weist einen Hauptreaktorbehälter auf. Innerhalb des Hauptreaktorbehälters ist ein Vorreaktorbehälter angeordnet. Hierdurch werden einerseits die in dem Biogasreaktor ablaufenden Reaktionen voneinander getrennt und andererseits eine indirekte Beheizung des Vorreaktorbehälters durch den Hauptreaktorbehälter gewährleistet.

Das Größenverhältnis dieser beiden Behälter zueinander richtet sich unter anderem nach den Verweilzeiten der Biomasse in den einzelnen Behältern. Als probates Fassungsvermögen des Hauptreaktorbehälters wird in der Druckschrift DE 202 19 144 U1 ein Gesamtvolumen von ca. 80 m³ angeführt, was einem Durchmesser von 4,0 m und einer Höhe von 7,5 m bis 8,0 m entspricht.

Auch die DE 34 11 264 A1 und EP 0 300 348 A2 offenbaren ebenfalls Größenverhältnisse in Biogasreaktoren.

Aus der DE 34 11 264 A1 geht hervor, dass der Durchmesser des Hauptgärbereichs vorteilhafterweise etwa 5 m betragen sollte.

Die EP 0 300 348 A2 offenbart, dass der Durchmesser eines Biogasreaktors im Bereich von 5 bis 15 m, vorzugsweise ca. 9 m, betragen kann. Die in der EP 0 300 348 A2 offenbarte Reaktorhöhe beträgt zwischen 15 und 50 m, vorzugsweise 30 m.

Das Reaktorbehältervolumen von ca. 80 m³ bildete im Stand der Technik gewissermaßen den Auftakt für größere Behältervolumina von zunächst 90 m³, dann 100 m³ und mittlerweile 110 m³. Reaktorbehälter dieser Größenordnungen befinden sich zur Zeit in Betrieb und erreichen Biogasvolumenströme zur Erzeugung von elektrischer Energie, die bei einem Behältervolumen von 90 m³ 50 kW, bei einem Behältervolumen von 100 m³ 55 kW und bei einem Behältervolumen von 110 m³ 60 kW beträgt. Die Volumenänderungen erfolgten hierbei über die Höhe, und zwar bei einem konstanten Behälterdurchmesser von ca. 3,5 m.

Diese Beispiele zeigen, dass den Behälterabmessungen bei der konstruktiven Ausgestaltung von Biogasreaktoren erhöhte Bedeutung zukommt. Hierbei sind allerdings auch gesetzliche Vorgaben zu berücksichtigen, die vorsehen, dass Biogasanlagen bis zu 75 kW elektrische Energie erzeugen, mindestens 80 Volumenprozent Gülle einsetzen müssen und eine gesonderte höhere Vergütung erhalten.

Es ist deshalb Aufgabe der Erfindung, einen Biogasreaktor der eingangs genannten Art zur Verfügung zu stellen, der bei einem Einsatz von 80 Volumenprozent Gülle elektrische Energie von mehr als 60 kW erzeugt.

Diese Aufgabe wird mit den Merkmalen des Anspruches 1 gelöst. Vorteilhafte Ausgestaltungen der Erfindung ergeben sich aus den Unteransprüchen.

Es hat sich gezeigt, dass mindestens 150 m³ Behältervolumen für den Hauptreaktorbehälter benötigt werden, um 75 kW elektrische Energie bei einem Einsatz von 80 % Gülle zu erzeugen. Durch die ermittelten Abmessungen werden für den Reaktor auch gesetzlichen Vorgaben Genüge geleistet, die den Einsatz von mindestens 80 Volumenprozent Gülle und die Erzeugung von elektrischer Energie bis zu 75 kW bei Biogasanlagen vorsehen.

Eine vorteilhafte Ausgestaltung der Erfindung sieht vor, dass eine in einen Prallteller mündende Steigumwälzleitung innerhalb des Behälters verläuft. Gegenüber bekannten Biogasreaktoren, bei denen der obere Teil der Steigumwälzleitung außerhalb des Reaktorbehälters verläuft, ist die Steigumwälzleitung gemäß der Erfindung komplett innenliegend verbaut. Hierdurch wird vorteilhafterweise erreicht, dass keine Wärme aus der Steigumwälzleitung nach außen tritt und somit keine Wärmeverluste auftreten.

Bei bekannten Biogasreaktoren erfolgt die Behälterbeheizung als Warmwasserheizung im Außenmantel, der als Doppelmantel ausgeführt ist. Ein Nachteil einer Warmwasserheizung im Außenmantel ist es jedoch, dass sich hierdurch der Innendurchmesser des Biogasreaktors und infolgedessen auch das Behältervolumen reduziert. Auch verursacht die Lösung einer Warmwasserheizung innerhalb des Außenmantels erhöhte Kosten, da ein Materialmehraufwand aufgrund einer weiteren Wandstärke im Gesamtumfang des Biogasreaktors erforderlich ist. In einer bevorzugten Ausführungsform der Erfindung weist daher der Biogasreaktor eine Heizvorrichtung auf, die zumindest zum Teil innerhalb des Reaktorbehälters angeordnet ist und der Steigumwälzleitung zugeordnet ist. Hierdurch wird vorteilhafterweise erreicht, dass im Vergleich zu einem Behälterdoppelmantel ca. 80 % Material eingespart werden kann.

Zweckmäßigerweise weist die Heizvorrichtung eine Doppelmantelrohrsteigleitung auf, die die Steigumwälzleitung umgibt. Durch diese konstruktive Ausgestaltung ist gewährleistet, dass das durch die Steigumwälzleitung geführte Substrat im Gegenstromverfahren kontinuierlich erwärmt wird. Hierzu ist vorgesehen, dass Heißwasser in einer Heizwasservorlaufleitung gepumpt und über eine Heizwasserrücklaufleitung, die die Steigumwälzleitung umgibt, wieder in ein Heizwasserreservoir zurückgeführt wird. Die Folge ist, dass sich ein Temperaturgradient zwischen Heizwasserrücklaufleitung und Steigumwälzleitung ergibt und somit ein Wärmeübertrag auf das Substrat in der Steigumwälzleitung erfolgt.

Eine praktikable Variante der Erfindung sieht vor, dass der Biogasreaktor einen Vorreaktorbehälter aufweist, der in Form eines Rohres vorliegt, wobei der Vorreaktorbehälter sich entlang der Reaktorwand zum Kopfbereich des Behälters hin erstreckt. Vorzugsweise beträgt der Durchmesser des Rohres zwischen 340 und 360 mm, besonders bevorzugt 350 mm.

Entgegen dem Stand der Technik liegt die Hydrolysestufe somit nicht in Gestalt eines Behälters vor, der zumindest zum Teil innerhalb des Reaktorbehälters angeordnet ist und dessen Inhalt über einen Siphon in den Hauptreaktorbehälter geleitet wird. Hierdurch werden vorzugsweise Verstopfungen innerhalb des Siphons vermieden. In erfindungswesentlicher Weise wird das Substrat im Rahmen der Hydrolysestufe durch das Rohr nach oben geführt und in den Hauptreaktorbehälter geleitet. Diese konstruktive Ausgestaltung bietet auch den Vorteil, dass bei Fehlen eines Siphons eine direkte Vermischung der Substrate in dem Hauptreaktorbehälter und in der Hydrolysestufe vermieden werden.

Es ist von Vorteil, wenn der Innendurchmesser des Hauptreaktorbehälters zwischen 3,6 m und 4,5 m, vorzugsweise 3,8 m, sowie die Höhe des Behälters zwischen 11,5 m und 15,5 m, vorzugsweise 13,5 m, beträgt.

Im Weiteren wird die Erfindung anhand der Zeichnung näher erläutert. Es zeigt in schematischer Darstellung:
- Fig. 1: einen Biogasreaktor gemäß der Erfindung und
- Fig. 2: eine perspektivische Darstellung der Doppelmantelrohrsteigleitung.

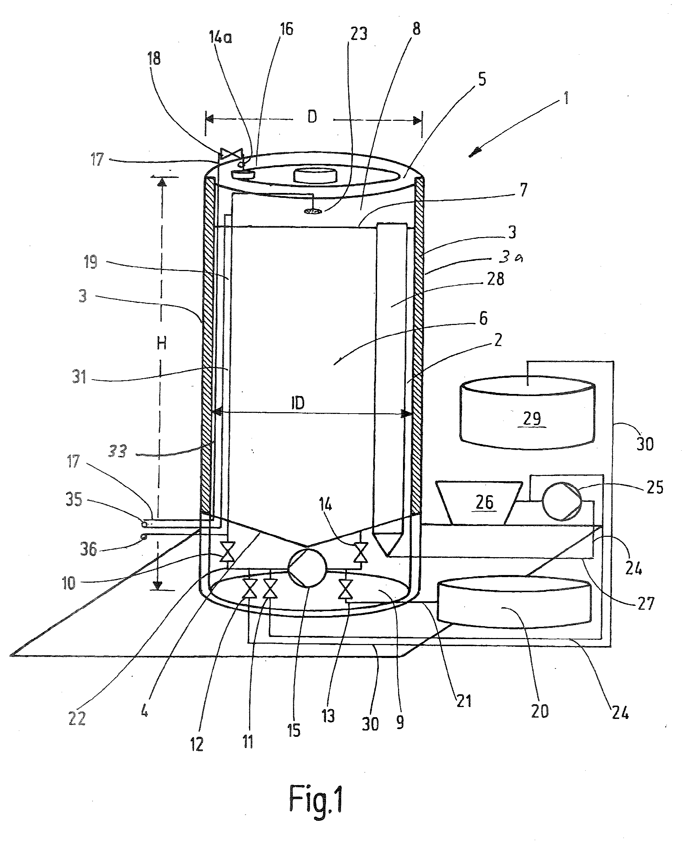

Fig. 1 zeigt einen erfindungsgemäßen Biogasreaktor, der mit dem Bezugszeichen 1 versehen ist.

Eine wesentliche Komponente des Biogasreaktors 1 ist der Hauptreaktorbehälter 2. Der Reaktorbehälter 2 ist mit dem Substrat 6 gefüllt. Oberhalb des Substratspiegels 7 befindet sich der Gasraum 8, der Bestandteil des Reaktorbehälters 2 ist. Der Reaktorbehälter 2 wird von der Wand 3 begrenzt. Oberseitig wird der Gasraum 8 bzw. der Reaktorbehälter 2 von dem Kopfbereich 5 begrenzt, der ein horizontales Bauteil ist und gleichzeitig den Biogasreaktor 1 nach oben abschließt. Unterseitig wird der Reaktorbehälter 2 von einem konisch ausgebildeten Boden 4 begrenzt.

Unterhalb des Bodens 4, d.h. im Fußbereich des Biogasreaktors 1, befindet sich der Reaktorraum 9, in dem verfahrenstechnische Vorrichtungen, wie Stoffschieber 10, 11, 12, 13, 14 und die Pumpe 15 untergebracht sind.

Das Reaktorvolumen, d.h. das Volumen des Reaktorbehälters 2 beträgt 150 m³. Der Innendurchmesser ID des Biogasreaktors 1 beträgt 3,8 m. Bei einer Dicke der Wand 3 sowie der Isolierung 3a und der in Fig. 1 nicht gezeigten Verkleidungen des Biogasreaktors 1 von insgesamt 0,4 m ergibt sich somit der Gesamtdurchmesser D des Biogasreaktors 1 von 4,2 m.

Die Höhe des Reaktorbehälters 2 einschließlich des Gasraumes 8 beträgt 14 m und die des Reaktorraumes 9 1,5 m, so dass eine Gesamthöhe H des Biogasreaktors 1 von 15,5 m resultiert. Die Höhe des Gasraumes beträgt 0,5 m.

Das in dem Gasraum 8 produzierte Biogas wird an einem Gasdom 16 im Kopfbereich 5 entnommen und über eine Gasleitung 17 abgeführt. Der Gasfluss kann mittels des Schiebers 18, der oberhalb des Gasdoms 16 angeordnet ist, unterbrochen werden. Nicht abführbares Biogas kann über die Überdrucksicherung 14a, die sich zwischen dem Gasdom 16 und dem Schieber 18 befindet, aus dem Gasraum 8 entweichen. Die Gasleitung 17 verläuft entlang der Wand 3 durch den Boden 4 in den Reaktorraum 9. Innerhalb des Reaktorraumes 9 zweigt die Gasleitung 17 ab und verläuft durch die Wand 3 nach außen.

Parallel zur Gasleitung 17 in dem Gasraum 8 und in dem Reaktorbehälter 2 verläuft die Steigumwälzleitung 19. Der Steigumwälzleitung 19 schließt sich die Saugleitung 22 an, die wiederum mit einer Leitung 21 verbunden ist, an der die Vorgrube 20 angeschlossen ist. Frischgülle wird aus der Vorgrube 20 über die Leitung 21, den Schieber 13, die Pumpe 15 sowie die Saugleitung 22 und den Schieber 10 in die Steigumwälzleitung 19 geführt und in den Kopfbereich 5 des Biogasreaktors 1 gepumpt. Die Steigumwälzleitung 19 mündet unterhalb des Kopfbereiches 5 in den Prallteller 23, so dass die Gülle auf das Substrat 6 verteilt wird. In erfindungswesentlicher Weise verläuft die in den Prallteller 23 mündende Steigumwälzleitung 19 innerhalb des Reaktorbehälters 2.

Zur Fütterung des Biogasreaktors 1 mit sogenannten Kosubstraten wird dem Biogasreaktor 1 ein Teil des Substrates 6 über die Saugleitung 22 entnommen. Mit Hilfe der in dem Reaktorraum 9 zwischen den Schiebern 10, 11, 12, 13, 14 angeordneten Pumpe 15 wird ein Teil des Substrates 6 über die Leitung 24 zur Substratmischpumpe 25 gepumpt. Die Substratmischpumpe 25 vermischt das Kosubstrat aus dem Behälter 26 mit dem aus dem Biogasreaktor 1 entnommenen Substrat und drückt dieses über die Leitung 27 in den Vorreaktorbehälter 28, der in Form eines Rohres vorliegt und sich entlang der Wand 3 zum Kopfbereich 5 des Reaktorbehälters 2 hin erstreckt. Das in dem Vorreaktorbehälter 28 befindliche Substrat/Kosubstratgemisch wird nach oben herausgedrückt und gelangt so in den Reaktorbehälter 2.

Dem Biogasreaktor 1 ist ein Endlager 29 zugeordnet, das über die Leitung 30 mit der Saugleitung 22 verbunden ist. Substrat 6 aus dem Reaktorbehälter 2 kann so über die Pumpe 15 und die Saugleitung 22 dem Reaktionsbehälter 2 entnommen und über die Leitung 30 ins Endlager 29 gepumpt werden. Hierdurch kann eine Entleerung des Reaktorbehälters 2 bis zu einem Minimalfüllstand vollzogen werden, wenn ein kritischer Wert überschritten wird. Der Füllstand wird dabei mit Hilfe des hydrostatischen Drucks sowie über einen Überfüllsensor und einen Sensor bestimmt, der den Minimalfüllstand darstellt.

Der Biogasreaktor 1 weist eine Heizvorrichtung 31 auf, die innerhalb des Reaktorbehälters 2 angeordnet ist und der Steigumwälzleitung 19 zugeordnet ist.

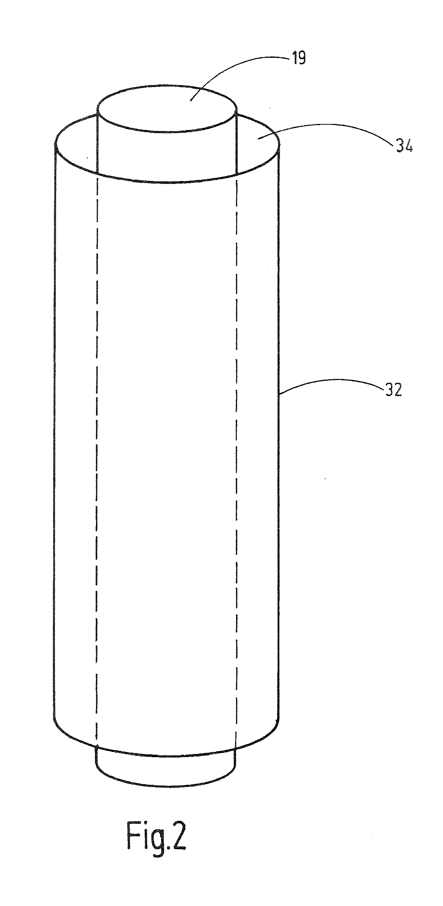

Die Heizvorrichtung 31 weist, wie aus Fig. 2 hervorgeht, eine Doppelmantelrohrsteigleitung 32 auf, die die Steigumwälzleitung 19 umgibt. Die Doppelmantelrohrsteigleitung 32 weist eine Heizwasserrücklaufleitung 34 auf. Um die Steigumwälzleitung 19 herum befindet sich die Heizwasserrücklaufleitung 34. Durch den in Fig. 1 gezeigten Eingang 35 wird Heizwasser in die Heizwasservorlaufleitung 33 gepumpt. Diese mündet unter der Reaktordecke in die Heizwasserrücklaufleitung 34, die sich im Doppelmantelbereich der Doppelmantelrohrsteigleitung 32 befindet.

Die Wärmeenergie des Heizwassers wird im Gegenstromverfahren auf die Steigumwälzleitung 19 bzw. auf das in der Steigumwälzleitung 19 geführte Substrat übertragen, so dass die Heizvorrichtung als Wärmetauscher fungiert. Über den in Fig. 1 gezeigten Ausgang 36 der Heizwasserrücklaufleitung 34 wird das Heizwasser abgeführt.

Die vorliegende Erfindung beschränkt sich in ihrer Ausführung nicht auf das vorstehend angegebene bevorzugte Ausführungsbeispiel. Vielmehr ist eine Anzahl von Varianten möglich, welche von der dargestellten Lösung auch bei grundsätzlich anders gearteten Ausführungen Gebrauch machen. Beispielsweise kann der Durchmesser 4,5 m und die Gesamthöhe H 17 m betragen.

### Bezugszeichenliste:

- 1: Biogasreaktor
- 2: Hauptreaktorbehälter
- 3: Wand
- 3a: Isolierung
- 4: Boden
- 5: Kopfbereich
- 6: Substrat
- 7: Substratspiegel
- 8: Gasraum
- 9: Reaktorraum
- 10, 11, 12, 13, 14: Stoffschieber
- 14a: Überdrucksicherung
- 15: Pumpe
- 16: Gasdom
- 17: Gasleitung
- 18: Schieber
- 19: Steigumwälzleitung
- 20: Vorgrube
- 21, 24, 27, 30: Leitung
- 22: Saugleitung
- 23: Prallteller
- 25: Substratmischpumpe
- 26: Behälter
- 28: Vorreaktorbehälter
- 29: Endlager
- 31: Heizvorrichtung
- 32: Doppelmantelrohrsteigleitung
- 33: Heizwasservorlaufleitung
- 34: Heizwasserrücklaufleitung
- 35: Eingang
- 36: Ausgang

## Patentansprüche

1. Biogasreaktor (1) mit einem Hauptreaktorbehälter (2),
**dadurch gekennzeichnet,**
**dass** der Durchmesser D des Reaktors (1) zwischen 4,2 m und 5 m und die Gesamthöhe H des Reaktors (1) zwischen 15,5 m und 18 m beträgt.

2. Reaktor nach Anspruch 1,
**dadurch gekennzeichnet,**
**dass** der Durchmesser D zwischen 4,4 m und 4,8 m und die Gesamthöhe H zwischen 16,5 m und 17,5 m beträgt.

3. Reaktor nach Anspruch 1 oder 2,
**dadurch gekennzeichnet,**
**dass** der Durchmesser D 4,5 m und die Gesamthöhe H 17 m beträgt.

4. Reaktor nach einem der Ansprüche 1 bis 3,
**dadurch gekennzeichnet,**
**dass** eine in einen Prallteller (23) mündende Steigumwälzleitung (19) vollständig innerhalb des Behälters (2) verläuft.

5. Reaktor nach einem der vorhergehenden Ansprüche,
**dadurch gekennzeichnet,**
**dass** er eine Heizvorrichtung (31) aufweist, die zumindest zum Teil innerhalb des Behälters (2) angeordnet ist und der Steigumwälzleitung (19) zugeordnet ist.

6. Reaktor nach Anspruch 5,
**dadurch gekennzeichnet,**
**dass** die Heizvorrichtung (31) eine Doppelmantelrohrsteigleitung (32) aufweist, die die Steigumwälzleitung (19) umgibt.

7. Reaktor nach einem der vorhergehenden Ansprüche,
**dadurch gekennzeichnet,**
**dass** er einen Vorreaktorbehälter (28) aufweist, der in Form eines Rohres vorliegt, wobei sich der Vorreaktorbehälter (28) entlang der Reaktorwand (3) zum Kopfbereich (5) des Hauptreaktorbehälters (2) hin erstreckt.

8. Reaktor nach Anspruch 7,
**dadurch gekennzeichnet,**
**dass** der Durchmesser des Rohres zwischen 340 und 360 mm, vorzugsweise 350 mm beträgt.

9. Reaktor nach einem der Ansprüche 1 bis 8,
**dadurch gekennzeichnet,**
**dass** der Innendurchmesser des Hauptreaktorbehälters (2) zwischen 3,6 m und 4,5 m sowie die Höhe des Behälters (2) zwischen 11,5 m und 15,5 m beträgt.

10. Reaktor nach Anspruch 9,
**dadurch gekennzeichnet,**
**dass** der Innendurchmesser des Hauptreaktorbehälters (2) 3,8 m und die Höhe des Behälters (2) 13,5 m beträgt.

11. Reaktor nach einem der vorhergehenden Ansprüche,
**dadurch gekennzeichnet,**
**dass** entlang der Wand (3) des Behälters (2) und innerhalb eines Reaktorraumes (9) eine Gasleitung (17) verläuft.
